# EUROPEAN PATENT APPLICATION

(11) **EP 4 406 930 A1**
(43) Date of publication of application: **31.07.2024**
(21) Application number: 23198240.6
(22) Date of filing: 19.09.2023
(51) Int. Cl.: C07B 59/00, C07D 209/42, G01N 30/72, G01N 33/68

(54) **ISOTOPE-LABELLED INDOLE CARBOXYLIC ACID COMPOUND, MIXTURES AND ANALYTICAL METHOD THEREWITH**

(30) Priority: 30.09.2022 JP 2022157405
(71) Applicant: JEOL Ltd., Akishima-shi, Tokyo 196-8558 (JP); Nihon University, Tokyo 102-8275 (JP)
(72) Inventor: KIKUTANI, Yoshikuni, Akishima, 1968558 (JP); TAKIWAKI, Masaki, Akishima, 1968558 (JP); UMEMURA, Hiroshi, Chiyoda-ku, 1028275 (JP); FUKUZAWA, Seketsu, Akishima, 1968558 (JP)
(74) Representative: Boult Wade Tennant LLP

(57) **Abstract**

The present invention is intended to provide a novel compound that can be used to detect or quantify a melanin-relating metabolite. The present invention provides a stable isotope-labeled indole carboxylic acid compound represented by Formula (I): 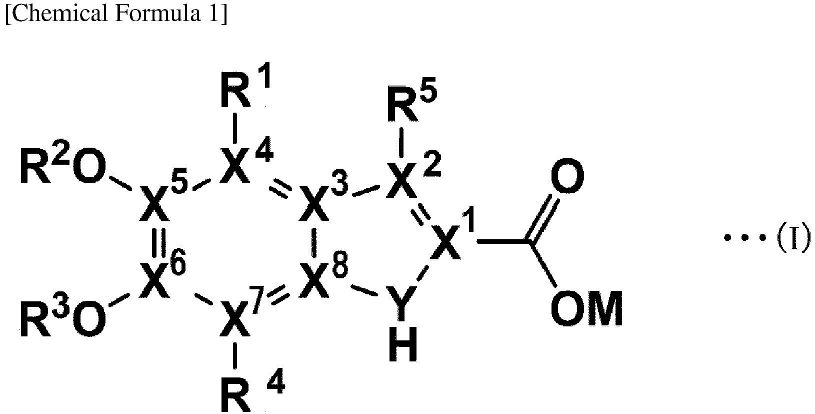 [in Formula (I), X¹ to X⁸ are each independently C or ¹³C; Y is N or ¹⁵N; M is H, Na, K, Li, CH₃, or C₂H₅; R¹ is H or D; R² is H, CH₃, ¹³CH₃, C₂H₅, or ¹³C₂H₅; R³ is H, CH₃, ¹³CH₃, C₂H₅, or ¹³C₂H₅; R⁴ is H or D; and R⁵ is H or D].

## Description

### Technical Field

The present invention relates to an indole carboxylic acid compound, a mixture of two or more indole carboxylic acid compounds, and an analytical method. The present invention specifically relates to a stable isotope-labeled indole carboxylic acid compound, a mixture of two or more stable isotope-labeled indole carboxylic acid compounds, and an analytical method using the compound or the mixture.

### Background Art

Melanin is a pigment synthesized in melanocytes. Melanin is a pigment synthesized in melanocytes and includes two types of melanin, eumelanin and pheomelanin. In the biosynthesis of them, various metabolites are produced. To measure such metabolites, some techniques have been developed.

For example, Non-Patent Document 1 discloses synthesis of deuterium-labeled eumelanin (precursor) metabolites. Non-Patent Document 2 discloses analysis of eumelanin-related indolic compounds in urine by HPLC with fluorometric detection. Non-Patent Document 3 discloses normal values of urinary excretion and serum concentration of 5-S-cysteinyldopa (hereinafter also referred to as 5-S-CD) and 6-hydroxy-5-methoxyindole-2-carboxylic acid (hereinafter also referred to as 6H5MI2C), biomarkers of melanoma progression.

### Citation List

### Non-Patent Document

[Non-Patent Document 1] Synthesis of deuterium-labeled eumelanin (precursor) metabolites. Pavel, S.; Muckiet, F. A. J. J. Label. Compd. Radiopharm. 1983, 20, 101-10.
[Non-Patent Document 2] Analysis of eumelanin-related indolic compounds in urine by high-performance liquid chromatography with fluorometric detection. J. Chromatogr. 1986, 375, 392-398.
[Non-Patent Document 3] Normal values of urinary excretion and serum concentration of 5-S-cysteinyldopa and 6-hydroxy-5-methoxyindole-2-carboxylic acid, biochemical markers of melanoma progression. Wakamatsu, K.; Ito, S.; Horikoshi, T. Melanoma Res. 1991, 1, 141-147.

### Summary of the Invention

### Technical Problem

Various metabolites involved in the above melanin synthesis may be usable as biomarkers for diseases or nutritional conditions. For example, these metabolites are probably used to understand the pathological condition of malignant melanoma, a type of skin cancer. For example, Non-Patent Document 3 discloses use of 5-S-CD and 6H5MI2C as biomarkers for melanoma, and a quantitative method by HPLC has been developed.

If such a metabolite can be detected or quantified with high sensitivity and high resolution, the metabolite probably has a higher utility value as a biomarker. Mass spectrometry, specifically, LC-MS/MS is a technique having high sensitivity and high resolution for compounds. To carry out the analytical method, an internal standard is required.

The present invention is therefore intended to provide a novel compound that can be used to detect or quantify a melanin-relating metabolite.

### Solution to Problem

The present invention provides a novel indole carboxylic acid compound. The compound can be used as an internal standard, for example, in mass spectrometry for detecting or quantifying melanin-relating metabolites.

The present invention provides the following aspects.
[1] A stable isotope-labeled indole carboxylic acid compound represented by Formula (I): 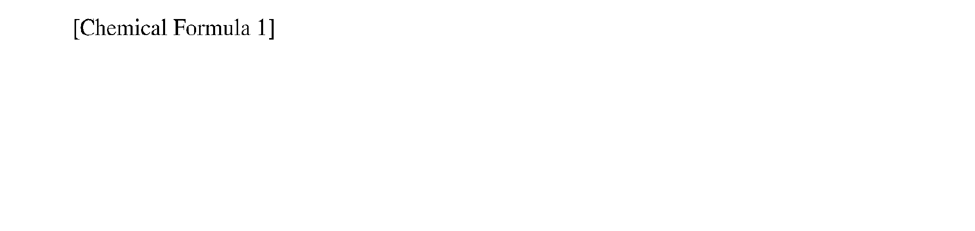 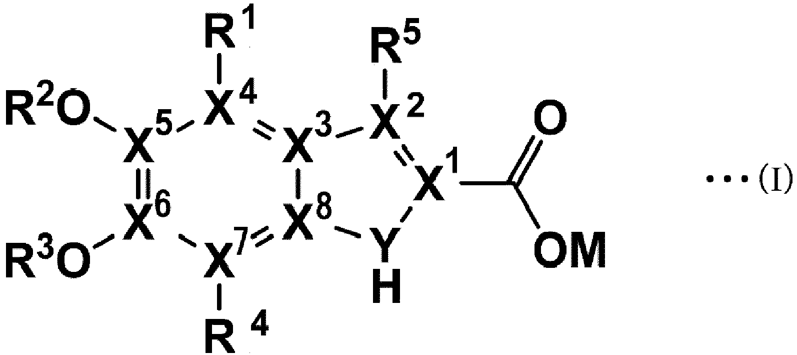 [in Formula (I),
   X¹ to X⁸ are each independently C or ¹³C;
   Y is N or ¹⁵N;
   M is H, Na, K, Li, CH₃, or C₂H₅;
   R¹ is H or D;
   R² is H, CH₃, ¹³CH₃, C₂H₅, or ¹³C₂H₅;
   R³ is H, CH₃, ¹³CH₃, C₂H₅, or ¹³C₂H₅;
   R⁴ is H or D; and
   R⁵ is H or D].
[2] The indole carboxylic acid compound according to the aspect [1], in which in Formula (I),
   R² is H, CH₃, or C₂H₅, and
   R³ is H, CH₃, or C₂H₅.
[3] The indole carboxylic acid compound according to the aspect [1], in which in Formula (I),
   R² is H, CH₃, or C₂H₅,
   R³ is H, CH₃, or C₂H₅, and
   one of R² and R³ is H.
[4] The indole carboxylic acid compound according to any one of the aspects [1] to [3], in which in Formula (I), X¹ to X⁸ are each C.
[5] The indole carboxylic acid compound according to any one of the aspects [1] to [4], in which in Formula (I), Y is N.
[6] The indole carboxylic acid compound according to any one of the aspects [1] to [5], in which one or both of R¹ and R⁴ are D.
[7] An analytical method using the indole carboxylic acid compound according to any one of the aspects [1] to [6].
[8] The analytical method according to the aspect [7], comprising carrying out mass spectrometry.
[9] The analytical method according to the aspect [8], in which in the mass spectrometry, the indole carboxylic acid compound is used as an internal standard.
[10] The analytical method according to the aspect [8] or [9], in which the mass spectrometry is carried out to detect or quantify an indole carboxylic acid compound in a biological sample.
[11] The analytical method according to the aspect [10], in which the indole carboxylic acid compound in a biological sample is a tumor marker for melanoma.
[12] A mixture of two or more different compounds that are each a stable isotope-labeled indole carboxylic acid compound represented by Formula (I): 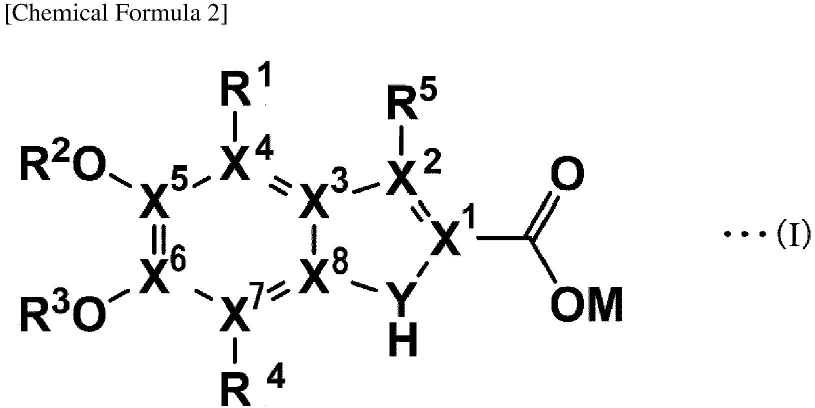 [in Formula (I),
   X¹ to X⁸ are each independently C or ¹³C;
   Y is N or ¹⁵N;
   M is H, Na, K, Li, CH₃, or C₂H₅;
   R¹ is H or D;
   R² is H, CH₃, ¹³CH₃, C₂H₅, or ¹³C₂H₅;
   R³ is H, CH₃, ¹³CH₃, C₂H₅, or ¹³C₂H₅;
   R⁴ is H or D; and
   R⁵ is H or D].
[13] The mixture according to the aspect [12], in which the mixture at least comprises an indole carboxylic acid compound of Formula (I) wherein R² is H and comprises an indole carboxylic acid compound of Formula (I) wherein R³ is H.
[14] An analytical method using the mixture according to the aspect [12] or [13].

### Advantageous Effects of Invention

According to the present invention, a standard usable to detect or quantify, for example, a melanin-relating metabolite is provided. The compound according to the present invention can be used as an internal standard, for example, in mass spectrometry for detecting or quantifying a melanin-relating metabolite. This enables detection or quantification of the metabolite with high sensitivity and high resolution.

The effect of the invention is not limited to that described in this paragraph and may be any of the effect described in the present specification.

### Brief Description of Drawings

[Fig. 1] Fig. 1 shows the biosynthetic pathway of melanin.
[Fig. 2] Fig. 2 shows a synthetic pathway for producing deuterium-labeled 6H5MI2C and 5H6MI2C.
[Fig. 3] Fig. 3 shows an NMR spectrum of 5H6MI2C-d₂ synthesized from L-dopa (Ring-d₃) as a starting material.
[Fig. 4] Fig. 4 shows an NMR spectrum of 6H5MI2C-d₂ synthesized from L-dopa (Ring-d₃) as a starting material.
[Fig. 5A] Fig. 5A shows an identification result by an LC/MS ESI negative scan of a mixture of 5H6MI2C-d₂ and 6H5MI2C-d₂.
[Fig. 5B] Fig. 5B shows an identification result by an LC/MS ESI negative scan of a mixture of 5H6MI2C-d₂ and 6H5MI2C-d₂.
[Fig. 6A] Fig. 6A shows a calibration curve of 5H6MI2C.
[Fig. 6B] Fig. 6B shows a calibration curve of 6H5MI2C.
[Fig. 7] Fig. 7 shows SRM chromatograms of 5H6MI2C and 6H5MI2C in a pooled serum.
[Fig. 8] Fig. 8 illustrates a method for producing the compound of the present invention.
[Fig. 9] Fig. 9 illustrates a method for producing the compound of the present invention.

### Description of Embodiments

Preferred embodiments of the present invention will be described below. However, the present invention is not limited to the following preferred embodiments, which can be arbitrarily changed within the scope of the invention.

### 1. Description of the present invention

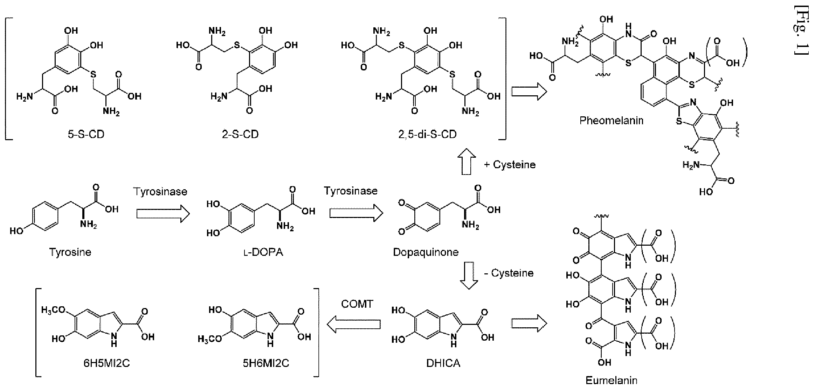

As described above, various metabolites are produced in the biosynthesis of melanin. The biosynthetic pathway of melanin is shown in Fig. 1. As shown in the figure, eumelanin and pheomelanin are derived from a common intermediate, dopaquinone that is biosynthesized by tyrosinase from tyrosine in melanocytes. When dopaquinone undergoes cysteine addition, dopaquinone is transformed through 5-S-CD, 2-S-CD, and 2,5-di-S-CD into pheomelanin. When dopaquinone does not undergo cysteine addition, dopaquinone is transformed through DHICA into eumelanin. Various metabolites derived from dopaquinone partially leak into the blood and are transformed in the liver by O-methyltransferase (COMT) into 6H5MI2C or 5H6MI2C, which is finally excreted into urine.

It has been proposed that some of these melanin-relating metabolites are used as a tumor marker for melanoma. For example, 5-S-CD may be clinically used as a tumor marker as disclosed in Non-Patent Document 3. Depending on the conditions of a human to be tested, however, the 5-S-CD value may not be abnormal even in a patient with tumor or may be false positive in some cases. In addition, 5-S-CD is rarely measured outside Japan. Improving the reliability of a tumor marker for melanoma should be useful.

The inventors of the present invention have developed a novel compound that can be used in mass spectrometry for detecting or quantifying a melanin-relating metabolite. Mass spectrometry using the novel compound enables detection or quantification of a melanin-relating metabolite with high sensitivity and high resolution.

Detecting or quantifying a melanin-relating metabolite with high sensitivity and high resolution as above contributes to the improvement in reliability of a melanoma examination technique.

Non-Patent Documents 2 and 3 disclose the detection of a melanin-relating metabolite by HPLC. However, each detection method disclosed in these documents detects fluorescence or electric conductivity derived from a catechol structure, and in the reliability on quantification, mass spectrometry is superior.

With the compound according to the present invention, a melanin-relating metabolite can be quantified by mass spectrometry. By mass spectrometry using the compound according to the present invention, a melanin-relating metabolite can be quantified with high accuracy. Such accurate quantification contributes to the improvement in reliability of a melanoma examination technique.

Non-Patent Document 1 discloses a stable isotope-labeled compound, but the total number of deuterium atoms introduced to one compound is 5. To synthesize the compound, a large amount of an expensive deuterium reagent is needed.

According to the present invention, a stable isotope-labeled compound to which a smaller number of deuterium atoms are introduced can be produced. In other words, in the production of the compound according to the present invention, an expensive deuterium reagent can be saved, and accordingly, a stable isotope-labeled compound can be synthesized at low cost.

According to the present invention, for example, in the clinical examination field, a trace metabolite can be detected by mass spectrometry with high sensitivity and can be quantified with high sensitivity. In addition, the present invention is applicable to any type of mass spectrometer. For example, to widely spread the quantification of a trace metabolite by mass spectrometry in the clinical examination field, it is desirable to yield the same value regardless of the time, the place, and the apparatus from any manufacturer. The compound according to the invention can be used in mass spectrometry regardless of the difference between apparatuses from various manufacturers. This can help the spread of quantification of a trace metabolite by mass spectrometry.

The present invention will next be described in further detail.

### 2. First embodiment (indole carboxylic acid compound)

The present invention provides an indole carboxylic acid compound represented by Formula (I): 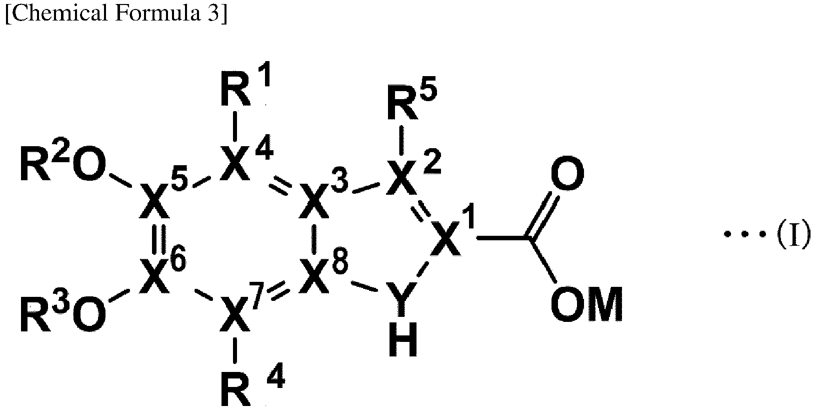 . Components of Formula (I) will next be described.

In Formula (I), X¹ to X⁸ are each independently C or ¹³C.

In other words, X¹ is C or ¹³C and is preferably C. X² is C or ¹³C and is preferably C. X³ is C or ¹³C and is preferably C. X⁴ is C or ¹³C and is preferably C. X⁵ is C or ¹³C and is preferably C. X⁶ is C or ¹³C and is preferably C. X⁷ is C or ¹³C and is preferably C. X⁸ is C or ¹³C and is preferably C.

From the viewpoint of the production cost, preferably, each of X¹ to X⁸ may be C.

In Formula (I), Y is N or ¹⁵N and is preferably N. From the viewpoint of the production cost, Y is preferably N.

In Formula (I), M is H, Na, K, Li, CH₃, or C₂H₅.

In an embodiment, M is H. In this case, examples of the compound include stable isotope-labeled 6H5MI2C or stable isotope-labeled 5H6MI2C. The structures of 6H5MI2C and 5H6MI2C are shown in Fig. 1.

In another embodiment, M is an alkali metal such as Na, K, and Li, and M is preferably Na or K. In the embodiment, M is a counter cation and can, for example, improve the solubility of the compound.

In yet another embodiment, M may be CH₃ or C₂H₅.

In Formula (I), R¹ is H or D; R⁴ is H or D; and R⁵ is H or D.

Preferably, one or two of R¹, R⁴, and R⁵ may be D, and the rest may be H. All of R¹, R⁴, and R⁵ may be D.

In an embodiment, in Formula (I), one or both of R¹ and R⁴ may be D. For example, in Formula (I), R¹ is D; R⁴ is D; and R⁵ is H. Alternatively, R¹ is D; R⁴ is H; and R⁵ is H. Alternatively, R¹ is H; R⁴ is D; and R⁵ is H.

In Formula (I), R² is H, CH₃, ¹³CH₃, C₂H₅, or ¹³C₂H₅, and R³ is H, CH₃, ¹³CH₃, C₂H₅, or ¹³C₂H₅. Preferably, one or both of R² and R³ are H.

In an embodiment, R² is H, CH₃, or C₂H₅, and R³ is H, CH₃, or C₂H₅. More preferably, R² is H or CH₃, and R³ is H or CH₃.

For example, R² is H, CH₃, or C₂H₅; R³ is H, CH₃, or C₂H₅; and one of R² and R³ may be H.

In a preferred embodiment, R² is H, and R³ is CH₃.

In another preferred embodiment, R² is CH₃, and R³ is H.

In yet another preferred embodiment, R² is H, and R³ is H.

The compound of the present invention has at least one stable isotope.

For example, the compound of the present invention may have at least one stable isotope, D (deuterium).

For example, the compound of the present invention may have at least one stable isotope, ¹³C.

For example, the compound of the present invention may have at least one stable isotope, ¹⁵N.

The position of such a stable isotope in the compound is as described above.

In a preferred embodiment, the number of stable isotopes, D, of the compound of the present invention is, for example, 3, 2, or 1. The stable isotope D may be present as R¹, R⁴, or R⁵ described above.

The number of stable isotopes, ¹³C, of the compound of the present invention is, for example, 12 or less, preferably 10 or less, and more preferably 8 or less. The stable isotope ¹³C may be present as X¹ to X⁸ described above and/or as a component of R² and/or R³.

In an embodiment, one or more of X¹ to X⁸ in Formula (I) may be a stable isotope ¹³C, and neither R² nor R³ has a stable isotope ¹³C (that is, R² and R³ are H, CH₃, or C₂H₅).

In another embodiment, the compound may have no stable isotope, ¹³C. In other words, X¹ to X⁸ may each be C, and R² and R³ may each be H, CH₃, or C₂H₅.

The number of stable isotopes, ¹⁵N, of the compound of the present invention is 1 or less. The stable isotope, ¹⁵N, may be present as Y described above.

In an embodiment, the stable isotope-labeled indole carboxylic acid compound of the present invention may be a compound represented by Formula (II).

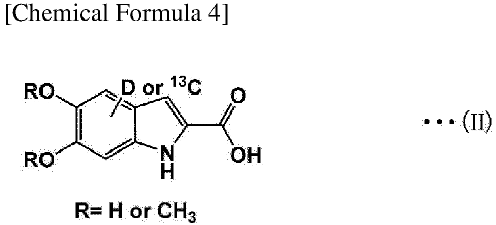

In Formula (II), two Rs are each independently H or CH₃ as described above. At least one of Hs and/or Cs constituting the indole ring may be a stable isotope D or a stable isotope ¹³C. Preferably, two Ds may be bonded to the six-membered ring (specifically at 4-position and 7-position) of the indole ring.

Preferably, the stable isotope-labeled indole carboxylic acid compound of the present invention may be a compound represented by Formula (III) or (IV). In the present specification, the compound of Formula (III) is also referred to as 6H5MI2C-4,7-d₂ or 6H5MI2C-d₂ as described later, and the compound of Formula (IV) is also referred to as 5H6MI2C-4,7-d₂ or 5H6MI2C-d₂.

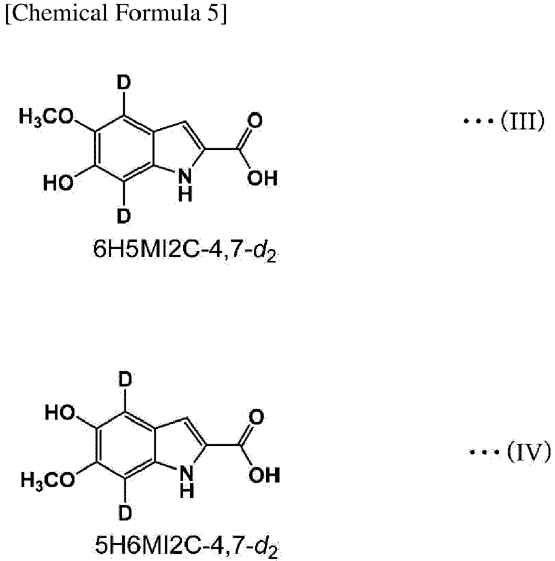

Of the melanin-relating metabolites, 6H5MI2C and 5H6MI2C are useful as tumor markers for melanoma. By mass spectrometry using the stable isotope-labeled compounds of Formulae (III) and (IV) as the internal standards, 6H5MI2C and 5H6MI2C can be accurately quantified, and the accurate quantification contributes to the usability of 6H5MI2C and 5H6MI2C as the tumor markers.

The compound of the present invention can be synthesized, for example, from a stable isotope-labeled L-DOPA as a starting material. Synthesis of the compound of the present invention using a stable isotope-labeled L-DOPA may be carried out by a technique known in the art and may be carried out, for example, as shown in Examples described later.

The stable isotope-labeled L-DOPA may be produced by a technique known in the art or is also commercially available. Synthesis of the stable isotope-labeled L-DOPA will be described below.

L-DOPA is synthesized, for example, from benzene, pyruvic acid, and ammonia. The synthesis may be carried out, for example, in accordance with the method described in Min, K.; Park, K.; Park, D-H.; Yoo, Y. J. Overview on the biotechnological production of L-DOPA. Appl. Microbiol. Biotechnol. 2015, 99, 575-584.

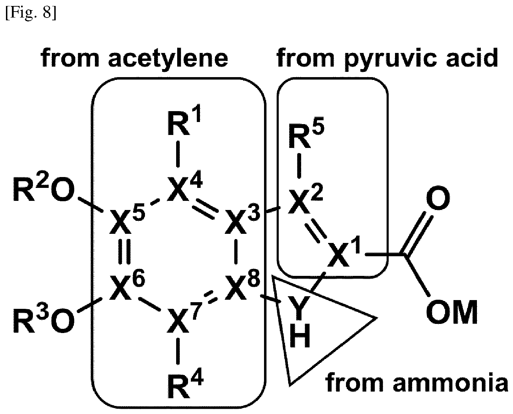

Of the compounds used in the synthesis of L-DOPA, the benzene may be synthesized, for example, from acetylene. Using a stable isotope-labeled acetylene as the acetylene yields a stable isotope-labeled benzene. For example, JP-A No. 2008-266149 discloses a production method of ¹³C-labeled benzene using a stable isotope-labeled acetylene, and such a production method may be used to prepare a stable isotope-labeled benzene. As described above, using a stable isotope-labeled acetylene enables introduction of stable isotopes to the six-membered ring moiety (X³ to X⁸) of Formula (I) as shown in Fig. 8.

For the pyruvic acid and the ammonia of the compounds used in the synthesis of L-DOPA, a stable isotope-labeled pyruvic acid and a stable isotope-labeled ammonia are commercially available. Alternatively, a stable isotope-labeled pyruvic acid and a stable isotope-labeled ammonia may be synthesized by a technique known in the art. Using the pyruvic acid enables introduction of stable isotopes to the five-membered ring moiety (X¹ and X²) in Formula (I) as shown in Fig. 8. Using the ammonia enables introduction of a stable isotope to the nitrogen (Y) in Formula (I) as shown in Fig. 8.

As described above, the carbons or the nitrogen constituting the skeleton of the compound of the Formula (I) may be stable isotope-labeled by appropriately using, as the material compounds, compounds that are stable isotope-labeled at corresponding elements.

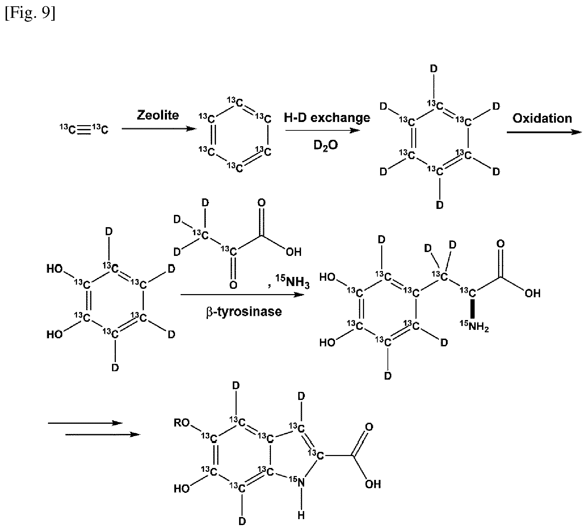

As for deuterium labeling (R¹, R⁴, and R⁵) in Formula (I), H-D exchange with deuterium oxide may be carried out, for example, as shown in Fig. 9.

For example, as shown in Fig. 9, reaction of a stable isotope-labeled acetylene under a zeolite catalyst enables synthesis of a stable isotope-labeled benzene. By subjecting the benzene to H-D exchange with deuterium oxide, the benzene is labeled with deuterium atoms. By subjecting the benzene to oxidation, hydroxy groups are introduced, and catechol is prepared. By reacting the catechol with a stable isotope-labeled pyruvic acid and a stable isotope-labeled ammonia in the presence of β-tyrosinase, a stable isotope-labeled L-DOPA is prepared as shown in the figure. By using the L-DOPA, the compound of the present invention may be synthesized.

### 3. Second embodiment (mixture)

The present invention also provides a mixture of two or more different indole carboxylic acid compounds of the stable isotope-labeled indole carboxylic acid compounds according to the present invention described in the above section 2.

In other words, the present invention also provides a mixture of two or more different compounds of the stable isotope-labeled indole carboxylic acid compounds represented by Formula (I): 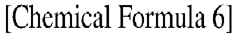 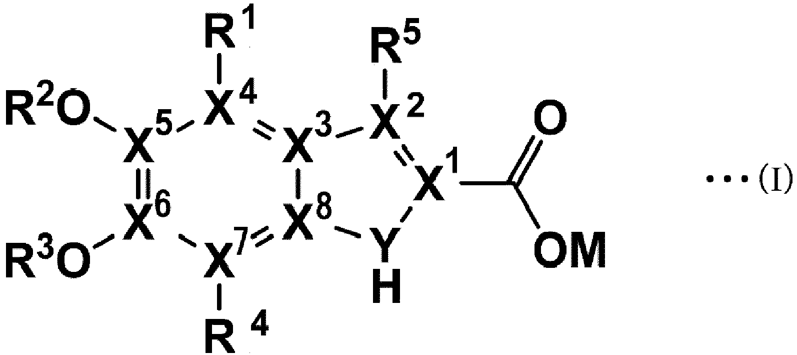 [in Formula (I),
X¹ to X⁸ are each independently C or ¹³C;
Y is N or ¹⁵N;
M is H, Na, K, Li, CH₃, or C₂H₅;
R¹ is H or D;
R² is H, CH₃, ¹³CH₃, C₂H₅, or ¹³C₂H₅;
R³ is H, CH₃, ¹³CH₃, C₂H₅, or ¹³C₂H₅;
R⁴ is H or D; and
R⁵ is H or D].

The description for Formula (I) in the section 2 is also applied to the present embodiment.

The mixture can also be used as an internal standard in mass spectrometry as described above. The two or more compounds contained in the mixture preferably have the same molecular weight. For example, the two or more compounds contained in the mixture may differ only in the structures of R² and R³ and have the same molecular weight.

Two or more indole carboxylic acid compounds having similar chemical structures (especially, two or more compounds having the same molecular weight) in a biological sample may not need to be detected or quantified separately and independently. In such a case, the internal standard may be such a mixture as described above to reduce cost.

The mixture may contain at least an indole carboxylic acid compound of Formula (I) in which R² is H and an indole carboxylic acid compound of Formula (I) in which R³ is H. For example, the mixture may be a mixture containing two compounds, a stable isotope-labeled 5H6MI2C and a stable isotope-labeled 6H5MI2C. For example, the mixture may be a mixture containing the compound of Formula (III) (6H5MIC-4,7-d₂) and the compound of Formula (IV) (5H6MI2C-4,7-d₂) and may specifically contain only these two compounds.

### 4. Third embodiment (analytical method)

The present invention also provides an analytical method using the indole carboxylic acid compound described in the above section 2 or using the mixture described in the above section 3. In the analytical method, the indole carboxylic acid compound or the mixture may be used as a standard and may specifically be used as an internal standard.

The analytical method may be an analytical method targeting an indole carboxylic acid compound identical with the indole carboxylic acid compound of the present invention described in the above section 2 except that the target compound is not labeled with any stable isotope. For example, the analysis target may be an indole carboxylic acid compound having, in place of the stable isotopes (D, ¹³C, and ¹⁵N) contained in the above indole carboxylic acid compound of the present invention, corresponding atoms (H, C, and N).

When the mixture is used, the analytical method may be an analytical method targeting an indole carboxylic acid compound identical with the indole carboxylic acid compound of the present invention described in the above section 2 except that the target compound is not labeled with any stable isotope. The analytical method may specifically be a mass spectrometry method. When the mixture is used, the analysis target may be at least one of the two or more indole carboxylic acid compounds according to the present disclosure contained in the mixture except that the target compound is not labeled with any stable isotope.

For example, when the mixture is a mixture containing the compound of Formula (III) (6H5MIC-4,7-d₂) and the compound of Formula (IV) (5H6MI2C-4,7-d₂), the analysis target may be one or both of 5H6MI2C and 6H5MI2C. In the analytical method, 5H6MI2C and 6H5MI2C may be analyzed without distinction.

The analytical method may comprise carrying out mass spectrometry. In the mass spectrometry, the analysis target may be detected or quantified while the indole carboxylic acid compound according to the present invention or the mixture according to the present invention is used as a standard (specifically an internal standard).

The mass spectrometry may detect or quantify an indole carboxylic acid compound, for example, in a biological sample. In particular, the mass spectrometry may be for detecting or quantifying an indole carboxylic acid compound labeled with no stable isotope in a biological sample. The indole carboxylic acid compound in a biological sample may, for example, be one or both of 5H6MI2C and 6H5MI2C.

In an embodiment, the indole carboxylic acid compound in a biological sample may be a tumor marker for melanoma. For example, 5H6MI2C and 6H5MI2C are promising as a tumor marker for melanoma. The analytical method of the present invention may comprise detecting or quantifying such a tumor marker for melanoma and may specifically comprise detecting or quantifying such a tumor marker for melanoma by mass spectrometry.

The analytical method of the present invention may comprise generating, on the basis of the detection result or the quantification result, data on the risk of melanoma in a human from which the biological sample is derived, data on the presence or absence of melanoma in a human from which the biological sample is derived, or data on the progression of melanoma in a human from which the biological sample is derived.

The mass spectrometry may comprise ionizing an analysis target. The ionization may be carried out, for example, by electrospray ionization (ESI), atmospheric pressure chemical ionization (APCI), or matrix-assisted laser desorption ionization (MALDI) and may be carried out by any ionization other than these ionizations.

The mass spectrometry may be carried out with a mass spectrometer used in the art and may be carried out, for example, with a liquid chromatography tandem mass spectrometer (LC-MS/MS) or a matrix-assisted laser desorption-ionization mass spectrometer (MALDI-MS), but the apparatus for carrying out the mass spectrometry is not limited to them.

A specific procedure of the mass spectrometry may be appropriately designed by a person skilled in the art, for example, according to the type of a sample.

### 5. Example

The present invention will next be described in more detail with reference to examples, but the present invention is not limited to these examples.

### [Example 1] Synthesis of deuterium-labeled 6H5MI2C and 5H6MI2C

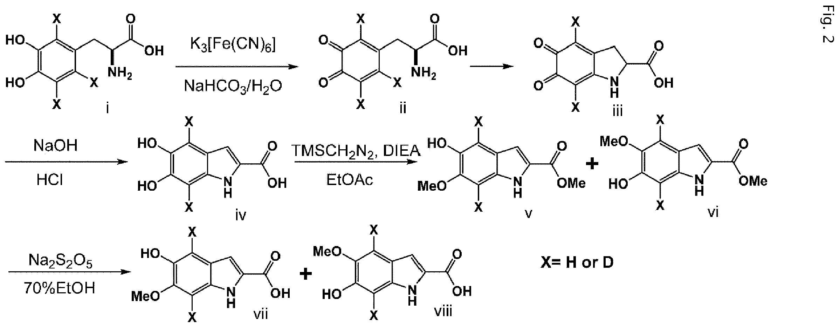

Deuterium-labeled 6H5MI2C and 5H6MI2C (i.e., the compounds of Formula (III) and Formula (IV)) were synthesized by the following procedure. The reaction pathway is shown in Fig. 2.

In a 100-mL eggplant flask, 0.1 g (0.5 mmol) of L-dopa (Ring-d₃, 98%, Cambridge Isotope Laboratories, Inc. MA, USA, Compound i in the figure) was placed, and 50 mL of distilled water was added. Under an inert gas atmosphere, the mixture was stirred to give an aqueous L-dopa (Ring-d₃). In 6 mL of distilled water, 660 mg of potassium hexacyanoferrate (III) (K₃[Fe(III)(CN)₆]) and 250 mg of sodium hydrogen carbonate (NaHCO₃) were dissolved. While the solution was stirred under an inert gas atmosphere, the whole quantity of the aqueous L-dopa (Ring-d₃) was added dropwise over 5 minutes. Immediately after completion of the dropwise addition, 7 mL of 1M aqueous sodium hydroxide was added, and the mixture was continuously stirred under an inert gas atmosphere for 15 minutes. To the mixture, 1.7 mL of 6M hydrochloric acid was added to quench the reaction. In this manner, Compound i was transformed to Compound iv as shown in the figure. In the figure, Compounds ii and iii are intermediates in the reaction.

Under an inert gas atmosphere, the reaction mixture was extracted with 25 mL of ethyl acetate three times. The obtained extract was washed with 10 mL of brine containing 19 mg of sodium pyrosulfite (Na₂S₂O₅) twice and was dried over magnesium sulfate. The magnesium sulfate was filtered off, and the filtrate was concentrated by using a rotary evaporator. The residue was dissolved in 2.5 mL of acetone, and 10 mL of hexane was added. After the removal of a brown oil, 15 mL of hexane was further added, followed by decantation to give a white precipitate. The resultant product was washed with hexane several times, and was dried under reduced pressure to give about 25 mg of 5,6-dihydroxyindole-2-carboxylic acid-d₂ (DHICA-d₂) (Compound iv in the figure).

About 20 mg of DHICA-d₂ was dissolved in 4 mL of a mixed solution of methanol and ethyl acetate (1:1). To the solution, 0.8 mL of trimethylsilyldiazomethane (10% hexane solution, ca 0.6M) was added twice, and the reaction mixture was allowed to stand in a sealed, light-shielded container under an inert gas atmosphere at room temperature for about 4 hours. A trace amount the reaction mixture was sampled every one hour, and the progress of methylation was monitored by LC-MS. The reaction was quenched by adding 0.1 mL of a mixed solution of methanol and formic acid (1:1), and the reaction mixture was transferred to a plurality of PP tubes and was dried by using a centrifugal evaporator. The solid obtained here is a mixture of methyl esters such as methyl 5,6-dihydroxyindole-2-carboxylate-d₂, methyl 5-hydroxy-6-methoxy-indole-2-carboxylate-d₂, methyl 6-hydroxy-5-methoxy-indole-2-carboxylate-d₂, and methyl 5,6-dimethoxyindole-2-carboxylate-d₂.

In the example, trimethylsilyldiazomethane was used as a methylating agent due to easy availability and handling, but another methylating agent such as diazomethane may be used.

The mixture was dissolved in ethyl acetate and was subjected to preparative thin-layer chromatography with chloroform as an eluent. To sufficiently separate bands corresponding to components, multiple development about five times with chloroform is required. Each component is a fluorescent substance and thus can be easily observed under an UV lamp. Each component is easily oxidized into dark brown, and thus the mixture is preferably developed in a light-shielded developing chamber purged with an inert gas. In addition, when the developing solvent is volatilized, inert gas purge is preferably carried out.

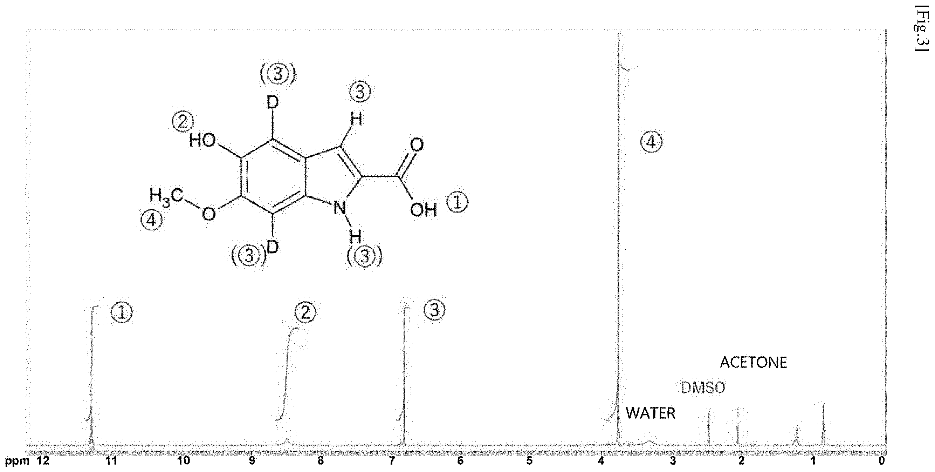

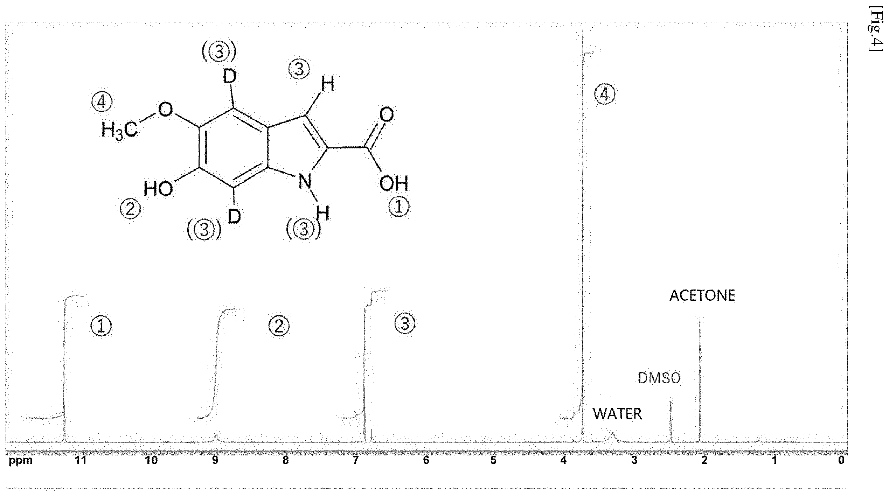

Identical component bands collected from a plurality of plates for thin-layer chromatography were extracted with ethyl acetate, and the extract was concentrated by using a rotary evaporator. Without further purification, methyl esters were hydrolyzed to give carboxylic acids. The methyl ester in this step is methyl 5-hydroxy-6-methoxyindole-2-carboxylate-d₂, methyl 6-hydroxy-5-methoxy-indole-2-carboxylate-d₂, or a mixture of these two esters at any ratio. To hydrolyze about 10 to 30 mg of the methyl esters, the methyl esters were placed in a 100-mL eggplant flask, and a solution of 380 mg of Na₂S₂O₅ in 6 mL of 70% ethanol was added. The mixture was stirred under an inert gas atmosphere at 37°C for 90 minutes, and then about 10 mL of 1M hydrochloric acid was added to adjust the pH to 1. The reaction mixture was extracted with 10 mL of ethyl acetate three times, and the combined extract was washed with 4 mL of brine twice. The washed liquid was dried over magnesium sulfate, and the magnesium sulfate was filtered off. The filtrate was concentrated by using a rotary evaporator. The product was recrystallized with acetone-hexane to give target compounds, 5-hydroxy-6-methoxy-indole-2-carboxylic acid-d₂ (Compound vii, X = D, in the figure) and 6-hydroxy-5-methoxy-indole-2-carboxylic acid-d₂ (Compound viii, X = D, in the figure). Fig. 3 and Fig. 4 show NMR spectra of 5H6MI2C-d₂ and 6H5MI2C-d₂ (solvent: deuterated DMSO).

Methylation with trimethylsilyldiazomethane or diazomethane yielded 5H6MI2C (5H6MI2C-d₂) two to three times more than 6H5MI2C (6H5MI2C-d₂). In some final applications (for example, for mass spectrometry), a mixture of 5H6MI2C-d₂ and 6H5MI2C-d₂ may be used, or the mixture may not interfere with an intended purpose. Such applications, for example, include a case in which the total amount of 5H6MI2C and 6H5MI2C in a sample is required to be determined and a case in which it is sufficient to detect the presence of one or both of 5H6MI2C and 6H5MI2C in a sample, but it is not necessary to specify which one is present.

In such a case, the methyl esters may not be exactly separated by thin-layer chromatography. In another case, a mixture of the methyl esters (optionally adjusted at an appropriate mixing ratio of, for example, 1:1 before hydrolysis) may be hydrolyzed in a single reaction container.

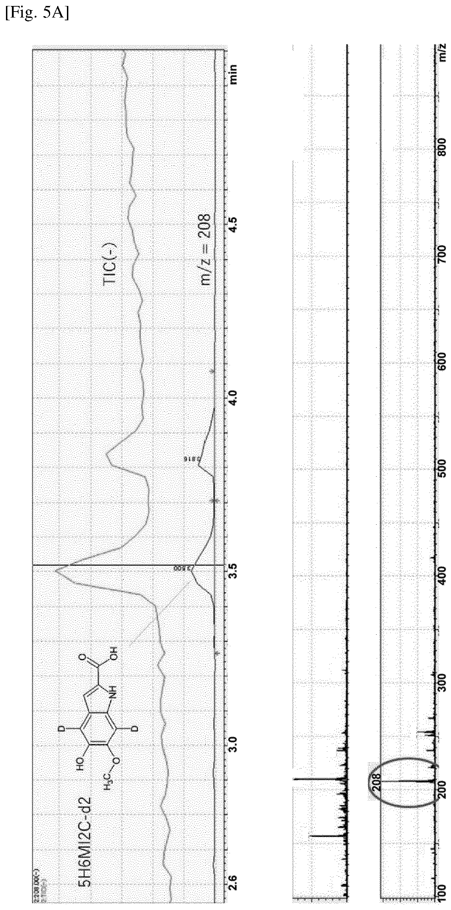

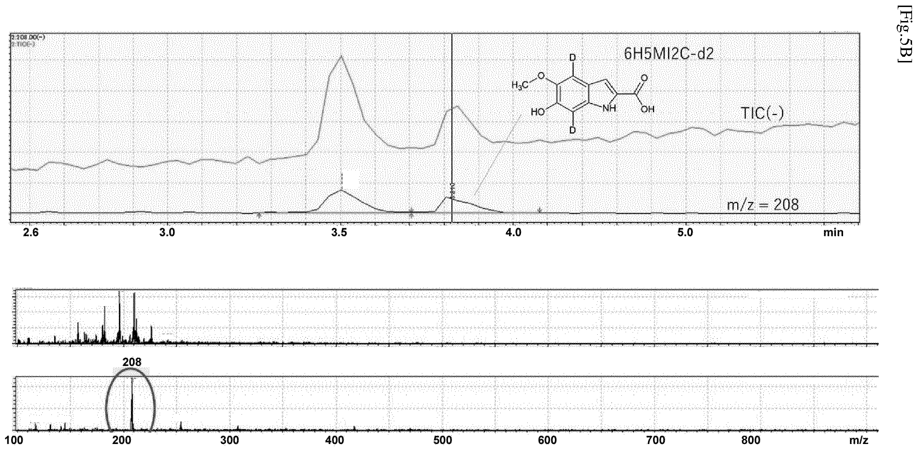

Fig. 5A and Fig. 5B show identification results by LC/MS ESI negative scan of the mixture of 5H6MI2C-d₂ and 6H5MI2C-d₂ synthesized by the above method. In each spectrum, a peak was observed at m/z = 208 that indicated that a proton was eliminated from the carboxylic acid.

### [Example 2] LC-MS/MS analysis of 6H5MI2C and 5H6MI2C in pooled serum

### (1) Preparation of calibrators

Authentic 5H6MI2C and 6H5MI2C were added to MSG3000 (Golden West Biologicals), which was a hormone-free serum, to prepare calibrators at concentrations shown in Table 1.

### [Table 1]

**Table 1: Calibrator concentration**

| | Cal. 1 | Cal. 2 | Cal. 3 | Cal. 4 | Cal. 5 | Cal. 6 | Cal. 7 | Cal. 8 |
|---|---|---|---|---|---|---|---|---|
| 5H6MI2C | 0.39063 | 0.78125 | 1.5625 | 3.125 | 6.25 | 12.5 | 25 | 50 |
| 6H5MI2C | 0.39063 | 0.78125 | 1.5625 | 3.125 | 6.25 | 12.5 | 25 | 50 |

### (2) Preparation of IS (internal standard) solution

A high concentration solution of 5H6MI2C-d₂ and 6H5MI2C-d₂ (1 µg/mL, 30% acetonitrile solution) was diluted with 4% phosphoric acid to prepare an internal standard solution at 5 ng/mL.

### (3) Sample pretreatment

Samples were pretreated in the following procedure.
1. SPE (solid phase extraction)
   1) 30 µL of a serum and 100 µL of the IS solution were mixed.
   2) 130 µL of the serum-IS mixed solution was loaded on an Oasis PRiME HLB 96-well µElution Plate (Water).
   3) 200 µL of 5% methanol was added to wash the plate.
   4) 100 µL of 95% methanol was added to elute the sample. This procedure was repeated twice.
2. Dryness: nitrogen blowing or centrifugal concentration for about 20 minutes
3. To the dried sample, 50 µL of 20% acetonitrile solution was added to prepare a sample for LC-MS/MS analysis.

### (4) LC analysis conditions

LC analysis conditions were as follows:
Apparatus: Waters, ACQUITY UPLC I-Class
Analytical column: Waters, ACQUITY UPLC BEH C18 1.7 mm I.D × 50 mm
Elution conditions: a flow rate of 0.4 mL/min; solvent A: 0.1% formic acid-water;
solvent B: 0.1% formic acid-acetonitrile
Detailed elution conditions were as shown in Table 2.

### [Table 2]

**Table 2: Elution conditions**

| Time (min) | 0 | 1 | 2.5 | 2.51 | 3.5 | 3.51 | 4.5 |
|---|---|---|---|---|---|---|---|
| B (%) | 10 | 10 | 30 | 95 | 95 | 10 | 10 |

### (5) MS/MS analysis conditions

MS/MS analysis conditions were as follows:
Apparatus: Waters, Xevo TQ-XS triple-quadrupole mass spectrometer
Ionization conditions: ESI negative ion mode
SRM parameters were as shown in Table 3.

### [Table 3]

**Table 3: SRM parameters**

| | SRM (m/z) | CE (eV) |
|---|---|---|
| 5H6MI2C | 206.3 > 147.1 | 18 |
| 5H6MI2C-d₂ | 208.3 > 149.1 | 18 |
| 6H5MI2C | 206.3 > 147.1 | 18 |
| 6H5MI2C-d₂ | 208.3 > 149.1 | 18 |

### • Analytical results

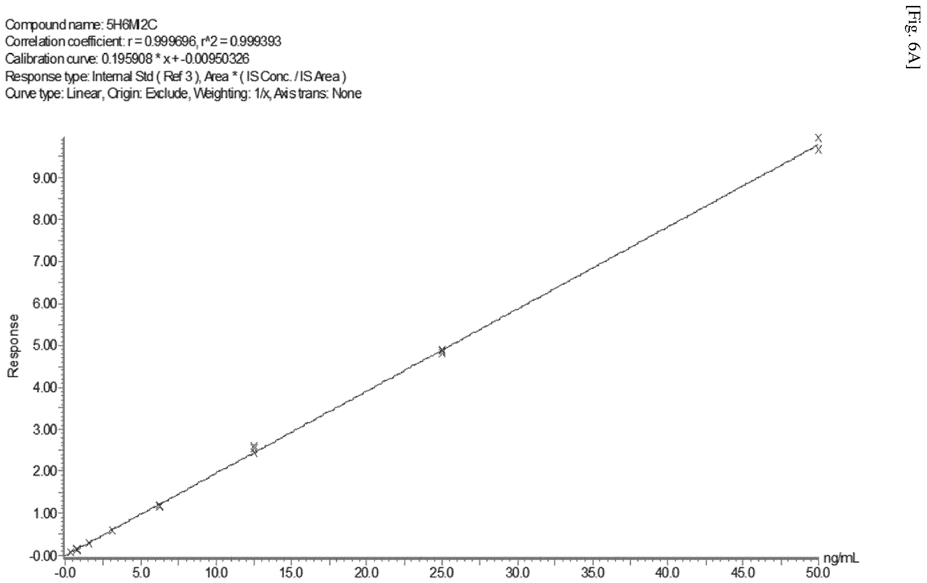

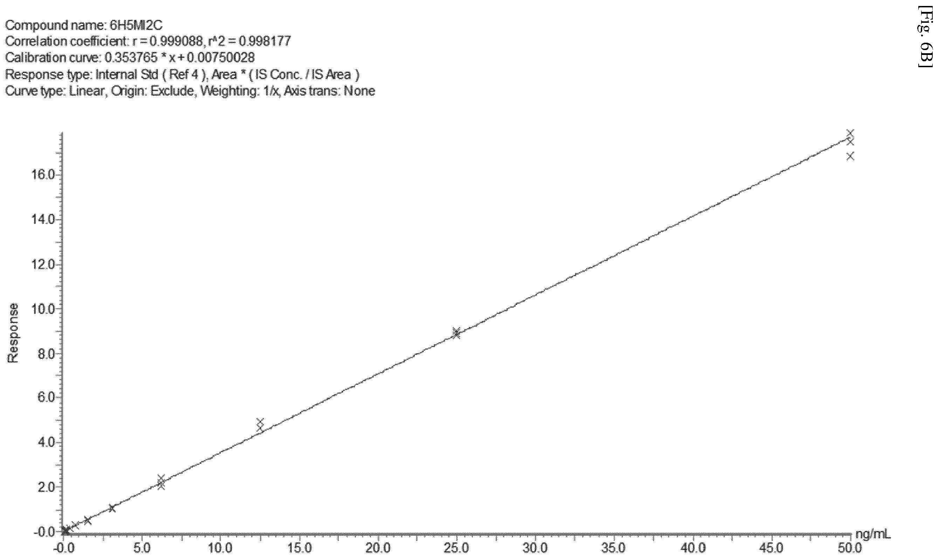

Fig. 6A and Fig. 6B show calibration curves of 5H6MI2C and 6H5MI2C.

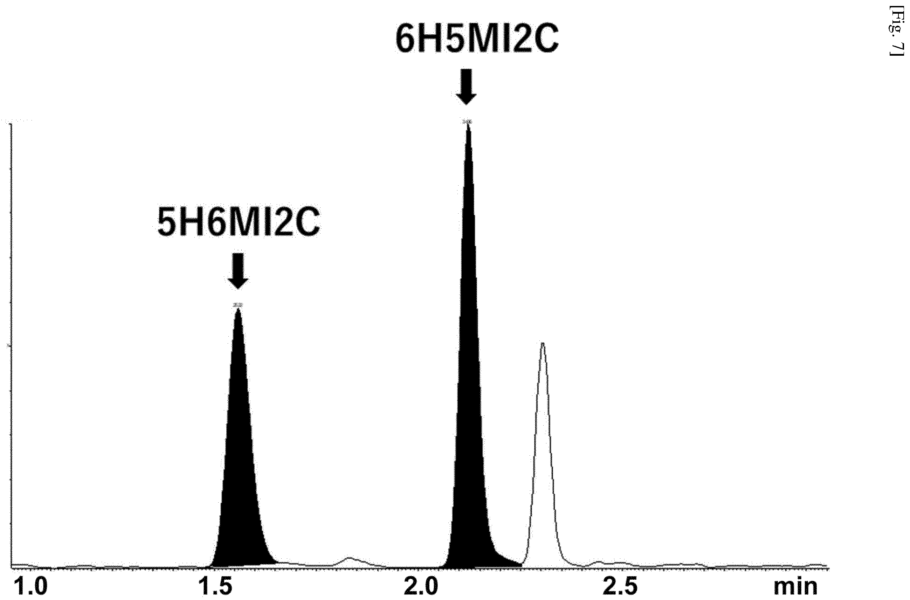

Fig. 7 shows an SRM chromatogram of 5H6MI2C and 6H5MI2C in a pooled serum.

### • Quantitative values of 5H6MI2C and 6H5MI2C in pooled serum

On the basis of the above analytical results, the concentration of 5H6MI2C and the concentration of 6H5MI2C in the pooled serum were quantified to be 0.41 ng/mL and 0.59 ng/mL, respectively.

In this manner, 5H6MI2C and 6H5MI2C can be quantified by mass spectrometry using the stable isotope-labeled indole carboxylic acid compounds according to the present invention. 5H6MI2C and 6H5MI2C are contained in extremely small amounts in a biological sample. Mass spectrometry using the stable isotope-labeled indole carboxylic acid compounds according to the present invention enables detection and quantification of such extremely small amounts of melanin-relating metabolites.

### [Measurement and analysis of clinical sample]

For about 80 clinical samples (samples with melanoma), 5H6MI2C and 6H5MI2C in a serum were quantified by the method described in Example 2, and the suitability of 5H6MI2C and 6H5MI2C as tumor markers was evaluated. The result revealed that these tumor markers increased as the disease progressed and reflect the disease status, and the compounds were sufficiently useful as the tumor markers.

## Claims

1. A stable isotope-labeled indole carboxylic acid compound represented by Formula (I): 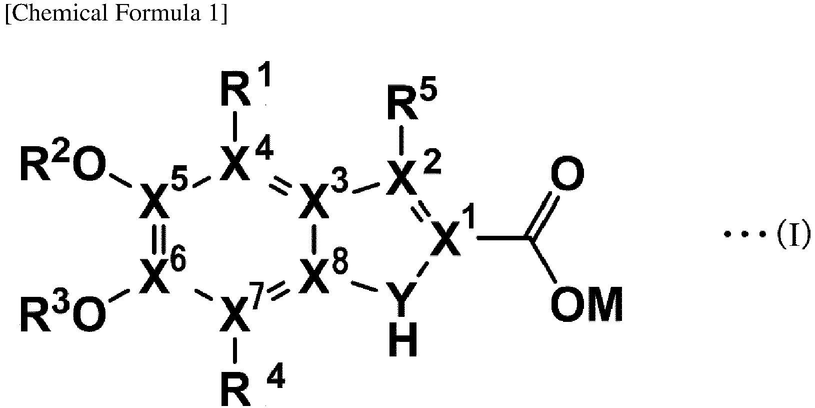 [in Formula (I),
X¹ to X⁸ are each independently C or ¹³C;
Y is N or ¹⁵N;
M is H, Na, K, Li, CH₃, or C₂H₅;
R¹ is H or D;
R² is H, CH₃, ¹³CH₃, C₂H₅, or ¹³C₂H₅;
R³ is H, CH₃, ¹³CH₃, C₂H₅, or ¹³C₂H₅;
R⁴ is H or D; and
R⁵ is H or D].

2. The indole carboxylic acid compound according to claim 1, wherein in Formula (I),
R² is H, CH₃, or C₂H₅, and
R³ is H, CH₃, or C₂H₅.

3. The indole carboxylic acid compound according to claim 1, wherein in Formula (I),
R² is H, CH₃, or C₂H₅,
R³ is H, CH₃, or C₂H₅, and
one of R² and R³ is H.

4. The indole carboxylic acid compound according to claim 1, wherein in Formula (I), X¹ to X⁸ are each C.

5. The indole carboxylic acid compound according to claim 1, wherein in Formula (I), Yis N.

6. The indole carboxylic acid compound according to claim 1, wherein one or both of R¹ and R⁴ are D.

7. An analytical method using the indole carboxylic acid compound according to claim 1.

8. The analytical method according to claim 7, comprising carrying out mass spectrometry.

9. The analytical method according to claim 8, wherein in the mass spectrometry, the indole carboxylic acid compound is used as an internal standard.

10. The analytical method according to claim 8, wherein the mass spectrometry is carried out to detect or quantify an indole carboxylic acid compound in a biological sample.

11. The analytical method according to claim 10, wherein the indole carboxylic acid compound in a biological sample is a tumor marker for melanoma.

12. A mixture of two or more different compounds that are each a stable isotope-labeled indole carboxylic acid compound represented by Formula (I): 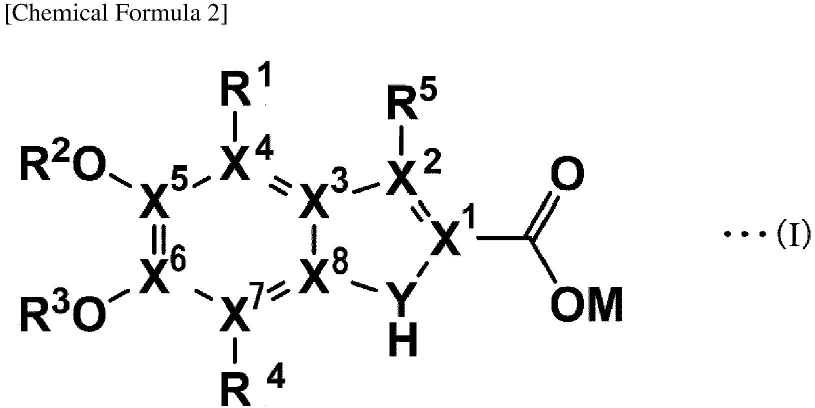 [in Formula (I),
X¹ to X⁸ are each independently C or ¹³C;
Y is N or ¹⁵N;
M is H, Na, K, Li, CH₃, or C₂H₅;
R¹ is H or D;
R² is H, CH₃, ¹³CH₃, C₂H₅, or ¹³C₂H₅;
R³ is H, CH₃, ¹³CH₃, C₂H₅, or ¹³C₂H₅;
R⁴ is H or D; and
R⁵ is H or D].

13. The mixture according to claim 12, wherein the mixture at least comprises
an indole carboxylic acid compound of Formula (I) wherein R² is H, and
an indole carboxylic acid compound of Formula (I) wherein R³ is H.

14. An analytical method using the mixture according to claim 12.
